# EUROPEAN PATENT SPECIFICATION

(11) **EP 1 502 956 B1**
(45) Date of publication and mention of the grant of the patent: **29.07.2009**
(21) Application number: 03728046.8
(22) Date of filing: 08.05.2003
(51) Int. Cl.: C12P 19/30

(54) **PROCESS FOR PRODUCING CYTIDINE 5'-DIPHOSPHATE CHOLINE**
VERFAHREN ZUR HERSTELLUNG VON CYTIDIN 5'-DIPHOSPHATCHOLIN
PROCÉDÉ DE PRODUCTION DE CYTIDINE 5'-DIPHOSPHATE CHOLINE

(30) Priority: 08.05.2002 JP 2002132917
(43) Date of publication of application: 02.02.2005
(73) Proprietor: Kyowa Hakko Bio Co., Ltd, Chiyoda-ku Tokyo 100-8185 (JP)
(72) Inventor: HASHIMOTO, Shin-ichi, Chiyoda-ku Tokyo 100-8185 (JP); ODA, Hideki, Hofu-shi, Yamaguchi 747-8522 (JP)
(74) Representative: Williams, Aylsa
(86) International application number: PCT/JP2003/005764
(87) International publication number: WO 2003/095660

(56) References cited:
- EP-A1- 0 553 821
- WO-A1-99/49073
- JP-A- 2001 238 691
- US-A1- 2002 037 573

## Description

### Technical Field

The present invention relates to a process for producing cytidine 5'-diphosphate choline.

### Background Art

Cytidine 5'-diphosphate choline (hereinafter abbreviated as CDP-choline) is a biosynthetic intermediate of phosphatidylcholine (lecithin), which is a phospholipid, and is useful for the treatment of head injuries, disturbance of consciousness following cerebral surgery, Parkinson's disease, post-apoplectic hemiplegia, etc.

Known methods for producing CDP-choline include chemical synthesis methods, methods for producing CDP-choline from cytidine 5'-triphosphate (hereinafter abbreviated as CTP), cytidine 5'-diphosphate (hereinafter abbreviated as CDP) or cytidine 5'-monophosphate (hereinafter abbreviated as CMP) using cells of microorganisms (Japanese Published Examined Patent Application Nos. 2358/73, 40758/73 and 2359/73), etc. However, these methods are problematic in that the yield is low, the starting materials are expensive, etc.

The methods using microorganisms so far reported include methods utilizing recombinant microorganisms and using, as starting materials, orotic acid, and choline or phosphorylcholine (Japanese Published Unexamined Patent Application No. 276974/93), uridine 5'-monophosphate (hereinafter abbreviated as UMP) and choline (or phosphorylcholine) (WO99/49073), and CMP and choline (Japanese Published Unexamined Patent Application No. 2001-103973). Orotic acid, which is relatively inexpensive, is an excellent material as a substrate for these reactions, but use of less expensive materials will make it possible to further lower the production cost. So far, there have been no reports of a method using uracil as a less expensive substrate.

### Disclosure of the Invention

An object of the present invention is to provide an efficient process for producing CDP-choline.

The present invention relates to a novel process for producing CDP-choline using inexpensive choline or phosphorylcholine, or a salt thereof, and uracil as substrates.

That is, the present invention relates to the following (1) to (3).
(1) A process for producing CDP-choline, which comprises contacting a biocatalyst having the activity to form CDP-choline from choline or phosphorylcholine, or a salt thereof, and uracil with choline or phosphorylcholine, or a salt thereof, and uracil in an aqueous medium; allowing the CDP-choline to form and accumulate in the aqueous medium; and recovering CDP-choline from the aqueous medium;
   and wherein the biocatalyst comprises cells selected from the group consisting of microorganisms belonging to the genera Escherichia or Corynebacterium, a culture of the cells or a treated matter of the cells;
   and wherein the cells are transformants obtainable by introducing DNA encoding cytidine 5'- triphosphate synthetase having the activity to form cytidine 5' - triphosphate from uridine 5'-triphosphate, DNA encoding choline kinase having the activity to form phosphorylcholine from choline, or DNA encoding cholinephosphate cytidyltransferase having the activity to form CDP-choline from cytidine 5' - triphosphate and phosphorylcholine.
(2) The process according to the above (1), wherein the microorganism belonging to the genus Corynebacterium is Corynebacterium ammoniagenes.
(3) The process according to the above (1), wherein the microorganism belonging to the genus Escherichia is Escherichia coli.

In the present invention, the biocatalysts have the activity to form CDP-choline from choline or phosphorylcholine, or a salt thereof, and uracil (hereinafter abbreviated as CDP-choline-forming activity).

The cells having the CDP-choline-forming activity are microorganisms belonging to the genera Escherichia or Corynebacterium.

Examples of the microorganisms belonging to the genus Escherichia are those belonging to Escherichia coli such as Escherichia coli MM294, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli GI698 and Escherichia coli TB1.

Examples of the microorganisms belonging to the genus Corynebacterium are those belonging to Corynebacterium glutamicum (e.g., Corynebacterium glutamicum ATCC 13032 and Corynebacterium glutamicum ATCC 13869), Corynebacterium ammoniagenes (e.g., Corynebacterium ammoniagenes ATCC 6872 and Corynebacterium ammoniagenes ATCC 21170) and Corynebacterium acteoacidophilum (e.g., Corynebacterium acetoacidophilum ATCC 13870).

It is possible to impart the CDP-choline-forming activity to cells which do not have the CDP-choline-forming activity by introducing DNA encoding an enzyme concerned with the CDP-choline-forming activity according to an ordinary method or by fusion with other cells having the activity and to use the obtained cells. Preferred are transformant obtained by introducing DNA encoding an enzyme concerned with the CDP-choline-forming activity into cells in the following manner.

The enzymes concerned with the CDP-choline-forming activity according to the present invention are cytidine 5'-triphosphate synthetase [EC 6.3.4.2] (PyrG) having the activity to form CTP from uridine 5'-triphosphate (UTP), choline kinase [EC 2.7.1.32] (CKI) having the activity to form phosphorylcholine from choline, cholinephosphate cytidyltransferase [EC 2.7.7.15] (CCT) having the activity to form CDP-choline from CTP and phosphorylcholine, etc.

DNAs encoding an enzyme concerned with the CDP-chollne-fomining activity are DNAs encoding the above enzymes PyrG, CKI or CCT.

DNA encoding PyrG has been cloned from the chromosome of Escherichia coli and the entire nucleotide sequence thereof has been determined [J. Biol. Chem., 261, 5568 (1986)]. An example of the source of DNA encoding PyrG is pMW6, which is a plasmid wherein a 2426-bp NruI-PstI fragment containing the DNA encoding PyrG derived from Escherichia coli is inserted at the SmaI-PstI site in the multicloning site of vector pUC8 of Escherichia coli [Gene, 19, 259 (1982)].

With respect to DNA encoding CCT, its entire nucleotide sequence has been determined [Eur. J. Biochem., 169, 477 (1987)]. An example of the source of DNA encoding CCT is plasmid pCC41 [Biochemistry, 60, 701 (1988)], which is a plasmid wherein a DraI fragment of 1296 base pairs (hereinafter abbreviated as bp) containing DNA encoding CCT derived from yeast is inserted at the SmaI site in the multicloning site of vector pUC18 of Escherichia coli [Gene, 33, 103 (1985)].

DNA encoding CKI has been similarly cloned from a yeast chromosome and its entire nucleotide sequence has been determined [J. Biol. Chem., 264, 2053 (1989)].An example of the source of DNA encoding CKI is plasmid pCK1D, which is a plasmid wherein a 2692-bp PstI-HindIII fragment containing DNA encoding CKI derived from yeast is inserted into the yeast-Escherichia coli shuttle vector YEpM4 [Mol. Cell. Biol., 7, 29 (1987)].

The biocatalyst having the CDP-choline forming-activity can be easily obtained by the following steps. On the basis of the nucleotide sequence information on the above DNAs encoding an enzyme concerned with the CDP-choline-forming activity, such DNA is obtained according to, for example, Molecular Cloning, A Laboratory Manual, Third Edition, Sambrook, et al. ed., Cold Spring Harbor Laboratory (2001) (hereinafter abbreviated as Molecular Cloning, Third Edition). The obtained DNA is inverted into an expression vector to prepare a recombinant DNA, which is used to transform the above cells as host cells. From the resulting transformant, the biocatalyst having the CDP-choline-forming activity can be obtained.

For example, DNA encoding PyrG, CCT or CKI is obtained from the above plasmid pMW6, pCC41 or pCK1D, and the obtained DNA is used to prepare a DNA fragment of an appropriate length containing a region encoding the polypeptide according to need.

If necessary, DNA wherein a nucleotide in the nucleotide sequence of the region encoding an enzyme concerned with the CDP-choline-forming activity is replaced so as to make a codon most suitable for the expression in a host cell is prepared. The DNA is useful for the efficient expression of an enzyme concerned with the CDP-choline-forming activity.

The DNA fragment or full length DNA encoding an enzyme concerned with the CDP-choline-forming activity is inserted downstream of a promoter in an appropriate expression vector to prepare a recombinant vector. The DNA fragments may be inserted into the respective expression vectors or plural DNAs may be inserted into one expression vector.

The recombinant vector is introduced into a host cell suited for the expression vector.

Examples of suitable host cells are the above-described cells.

The useful expression vectors are those capable of replicating autonomously or integrating into the chromosome in the host cells and comprising a promoter at a position appropriate for transcribing the DNA encoding

For the bacterium used as the host cell, it is preferred that the recombinant vector comprising the DNA encoding the polypeptide of the present invention is capable of replicating autonomously in the procaryote and comprises a promoter, a ribosome binding sequence, the DNA of the present invention and a transcription termination sequence. The recombinant vector may further comprise a gene regulating the promoter.

Suitable expression vectors include pBTrp2, pBTac1 and pBTac2 (all available from Boehringer Mannheim), pKK233-2 (Pharmacia), pSE280 (Invitrogen), pGEMEX-1 (Promega), pQE-8 (QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pTrs30 [prepared from Escherichia coli JM109/pTrS30 (FERM BP-5407)], pTrs32 [prepared from Escherichia coli JM109/pTrS32 (FERM BP-5408)], pGHA2 [prepared from Escherichia coli IGHA2 (FERM BP-400), Japanese Published Unexamined Patent Application No. 221091/85], pGKA2 [prepared from Escherichia coli IGKA2 (FERM BP-6798), Japanese Published Unexamined Patent Application No. 221091/85], pTerm2 (US4686191, US4939094, US5160735), pSupex, pUB110, pTP5, pC194, pEG400 [J. Bacteriol., 172, 2392 (1990)], pGEX (Pharmacia) and pET system (Novagen).

As the promoter, any promoters capable of functioning in host cells can be used. For example, promoters derived from Escherichia coli or phage, such as trp promoter (Pₜᵣₚ), lac prompter, P_{L} promoter, P_{R} promoter and T7 promoter can be used. Artificially designed and modified promoters such as a promoter in which two Ptrps are combined in tandem (Pₜᵣₚ x 2), tac promoter, lacT7 promoter and let I promoter, etc. can also be used.

It is preferred to use a plasmid in which the distance between the Shine-Dalgarno sequence (ribosome binding sequence) and the initiation codon is adjusted to an appropriate length (e.g., 6 to 18 nucleotides)

In the recombinant vector of the present invention, the transcription termination sequence is not essential for the expression of the DNA encoding an enzyme concerned with the CDP-choline-forming activity, but it is preferred to place the transcription termination sequence immediately downstream of the structural gene.

Introduction of the recombinant vector can be carried out by any of the methods of introducing DNA into the above host cells, for example, the method using calcium ion [Proc. Natl. Acad. Sci. USA, 69, 2110 (1972)], the protoplast method (Japanese Published Unexamined Patent Application No. 248394/88) and the methods described in Gene, 17, 107 (1982) and Molecular & General Genetics, 168, 111 (1979).

Two or more different kinds of cells may be appropriately combined to obtain the CDP-choline-biosynthesizing activity and used as the cells having the CDP-choline-biosynthesizing activity.

The above DNA encoding an enzyme concerned with the CDP-choline-forming activity may be introduced into the cell according to a conventional method, whereby a cell having enhanced CDP-choline-forming activity can be obtained.

Also in the case of cells having the CDP-choline-forming activity, two or more different kinds of cells can be used in combination.

An example of the combination is that of a microorganism belonging to the genus Corynebacterium and a microorganism belonging to the genus Escherichia.

Any of natural media and synthetic media can be used as the medium for culturing the cells insofar as it is a medium suitable for efficient culturing of the cells which contains carbon sources, nitrogen sources, inorganic salts, etc. which can be assimilated by the cells.

As the carbon sources, any carbon sources that can be assimilated by the cells can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

Examples of the nitrogen sources include ammonia, ammonium salts of organic or inorganic acids such as ammonium chloride, ammonium sulfate, ammonium acetate and ammonium phosphate, other nitrogen-containing compounds, peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is carried out under aerobic conditions, for example, by shaking culture or submerged spinner culture under aeration. The culturing temperature is preferably 15 to 50°C, and the culturing period is usually 16 hours to 7 days. The pH is preferably maintained at 3.0 to 9.0 during the culturing. The pH adjustment is carried out by using an organic or inorganic acid, an alkali solution, urea, calcium carbonate, aqueous ammonia, etc.

When the cell is a transformant and the recombinant DNA used for transformation carries an antibiotic resistance gene, an antibiotic corresponding to the antibiotic resistance gene carried by the recombinant DNA may be added to the medium for culturing the cell.

When two or more different kinds of cells, cultures of the cells or treated matters of the cells are used as the biocatalyst, those which are obtained by culturing these cells separately or in the same medium according to the above methods can be used.

When two or more different kinds of cells are cultured in the same medium, these cells can be cultured simultaneously, or during or after the culturing of one kind of cells, the other kind of cells may be added to the medium for culturing.

Combinations of two or more different kinds of cells may be made among any cells selected from the group defined abov.e

Examples of the combinations of a microorganism and a microorganism include any combinations of the above microorganisms, specifically, combinations of the microorganisms belonging to the genera selected from the group consisting of the genera Escherichia and Corynebacterium.

The treated matters of the above cells used as the biocatalyst having the CDP-choline-forming activity include concentrates or dried matters of the above-obtained culture of the cells obtained by concentration or drying of the culture of the cells with a concentrator or a drier, cells obtained by subjecting the culture of the cells to solid-liquid separation by filtration or centrifugation, dried matters of the cells obtained by drying the cells with a drier , etc., surfactant- or organic solvent-treated matters of the cells obtained by treating the cells with a surfactant or an organic solvent, and cytolytic enzyme-treated matters of the cells obtained by treating the cells with a cytolytic enzyme such as lysozyme.

Crude enzymes or purified enzymes obtained by subjecting the above treated matters of the cells to ordinary methods for purification of enzymes such as salting-out, isoelectric precipitation, precipitation with an organic solvent, dialysis and various chromatographies can also be used as the treated matters of the cells.

When two or more kinds of cells are used, the treated matters separately prepared from two or more kinds of cells may be separately used as the biocatalysts having the CDP-choline-biosynthesizing activity, or a mixture of these treated matters may be used as the biocatalyst having the CDP-choline-biosynthesizing activity.

The above cells or treated matters thereof may also be used as the biocatalyst after being immobilized on a water-insoluble carrier or gel

The process for producing CDP-choline is described in detail below.

CDP-choline can be produced by contacting the above biocatalyst with substrates in an aqueous medium, allowing CDP-choline to form and accumulate in the aqueous medium, and recovering CDP-choline, from the aqueous medium.

Specifically, the above biocatalyst is mixed with choline or phosphorylcholine, or a salt thereof, and uracil as substrates in an aqueous medium, and the resulting mixture is, if necessary with addition of other components, allowed to stand at 20 to 50°C for 2 to 48 hours, during which the pH was maintained at 5 to 10, preferably 6 to 8.

The amount of the biocatalyst to be used varies depending upon the specific activity, etc. of the biocatalyst. For example, when a culture or treated matter of the cells is used as the biocatalyst, it is appropriate to use it in an amount of 5 to 500 mg, preferably 10 to 300 mg (in terms of wet cells obtained by centrifuging the culture or treated matter of the cells) per mg of uracil.

Choline, phosphorylcholine and their salts include choline, choline halides such as choline chloride, choline bromide and choline iodide, choline bicarbonate, choline bitartrate, methylcholine sulfate, choline dihydrogen citrate, phosphorylcholine, and phosphorylcholine halides such as phosphorylcholine chloride. Preferred are halides of choline or phosphorylcholine, among which choline chloride and phosphorylcholine chloride are further preferably used.

Choline or phosphorylcholine, or a salt thereof, and uracil can be obtained by chemical synthesis or by fermentation using microorganisms. They are not necessarily highly purified and may contain impurities such as salt insofar as the impurities do not inhibit CDP-choline-forming reaction. Commercially available ones may also be used.

The concentration of choline or phosphorylcholine, or a salt thereof, and uracil is preferably 1 mmol/l to 1 mol/l, more preferably 10 to 200 mmol/l.

Other components to be added according to need include energy donors, phosphate ions, magnesium ions, ammonium ions, surfactants and organic solvents. These components need not be added when they are supplied in sufficient amounts from the biocatalyst, etc.

Examples of the energy donors are carbohydrates (e.g., glucose, fructose and sucrose), molasses, starch hydrolyzate, organic acids (e.g., pyruvic acid, lactic acid, acetic acid and α-ketoglutaric acid) and amino acids (e.g., glycine, alanine, aspartic acid and glutamic acid). The energy donor is preferably used at a concentration of 0.02 to 2.0 mol/l.

As the phosphate ion, orthophosphoric acid, pyrophosphoric acid, polyphosphoric acids (e.g., tripolyphosphoric acid and tetrapolyphosphoric acid), polymetaphosphoric acid, inorganic phosphates (e.g., potassium phosphate, dipotassium hydrogenphosphate, sodium dihydrogenphosphate and disodium hydrogenphosphate), etc. can be used. The phosphate ion is preferably used at a concentration of 10 to 500 mmol/l.

As the magnesium ion, inorganic magnesium salts (e.g., magnesium sulfate, magnesium nitrate and magnesium chloride), organic magnesium salts (e.g., magnesium citrate), etc. can be used. The magnesium ion is preferably used at a concentration of 5 to 200 mmol/l.

As the ammonium ion, those contained in aqueous ammonia, ammonia gas, various inorganic or organic ammonium salts, yeast extract, corn steep liquor, etc. can be used. Organic nutrient sources containing ammonium ions such as glutamine and Casamino acid can also be used as the ammonium ion. The ammonium ion is preferably used at a concentration of 10 mmol/l to 2 mol/l.

As the surfactant, any surfactants that promote CDP-choline formation can be used. Examples of suitable surfactants include anionic surfactants such as sodium dioctyl sulfosuccinate (e.g., Rapisol B-80, NOF Corporation) and lauroyl sarcosinate, nonionic surfactants such as polyoxyethylene cetyl ether (e.g., Nonion P-208, NOF Corporation) and tertiary amines such as alkyldimethylamine (e.g., Tertiary Amine FB, NOF Corporation). The surfactant is used usually at a concentration of 0.1 to 100 g/l, preferably 1 to 50 g/l.

Examples of suitable organic solvents are xylene, toluene, aliphatic alcohols (e.g., methyl alcohol, ethyl alcohol and butyl alcohol), acetone, ethyl acetate and dimethyl sulfoxide. The organic solvent is used usually at a concentration of 0.1 to 100 ml/l, preferably 1 to 50 ml/l.

Suitable aqueous media include water and buffers such as phosphate buffer, HEPES (N-2-hydroxyethylpiperazine-N-ethanesulfonic acid) buffer, Tris [Tris(hydroxymethyl)aminomethane] hydrochloride buffer.

Further, a medium for culturing the cells used as the biocatalyst, a culture of the cells and a supernatant of the culture can also be used as the aqueous medium. When the medium, culture, culture supernatant, etc. are used as the aqueous medium and the cells are used as the biocatalyst, the cells may be contacted with choline or phosphorylcholine, or a salt thereof, and uracil during the culturing or after the completion of the culturing. The medium for the cells and the culturing conditions are the same as those described above.

In this manner, CDP-choline can be formed and accumulated in the aqueous medium. The formed and accumulated CDP-choline can be isolated and purified by ordinary methods for isolation and purification such as ion exchange chromatography, adsorption chromatography and salting-out.

Certain embodiments of the present invention are illustrated in the following examples.

### Best Modes for Carrying Out the Invention

### Example 1

Escherichia coli MM294/pCKG55 (Japanese Published Unexamined Patent Application No. 276974/93, FERM BP-3717), which is a strain obtained by introducing plasmid pCKG55 carrying the CCT gene and the CKI gene derived from yeast and the PyrG gene into Escherichia coli MM294, was inoculated into 200 ml of L medium [10 g/l Bacto-tryptone (Difco), 5 g/l yeast extract (Difco) and 5 g/l sodium chloride, pH 7.2] containing 50 mg/l ampicillin in a 2-1 Erlenmeyer flask with baffles, followed by rotary shaking culture at 220 rpm at 25°C for 24 hours. The obtained culture (20 ml) was inoculated into 2.5 1 of a liquid medium comprising 5 g/l glucose (separately sterilized), 5 g/l peptone (Kyokuto Pharmaceutical Ind. Co., Ltd.), 6 g/l disodium phosphate, 3 g/l potassium dihydrogenphosphate, 1 g/l ammonium chloride, 250 mg/l magnesium sulfate heptahydrate (separately sterilized) and 4 mg/l vitamin B₁ (separately sterilized) (pH unadjusted) in a 5-1 fermentor, followed by culturing at 28° C with stirring (600 rpm) and aeration (2.5 l/minute). The pH was maintained at 7.0 with 14% (v/v) aqueous ammonia through the culturing.

During the culturing, the culture was sampled and the glucose concentration in the supernatants obtained by centrifuging the samples was measured. When the glucose concentration in the supernatant became zero, 250 ml of the culture was aseptically recovered.

The recovered culture was inoculated into 2.5 1 of a liquid medium comprising 5 g/l glucose (separately sterilized), 5 g/l peptone (Kyokuto Pharmaceutical Ind. Co., Ltd.), 6 g/l disodium phosphate, 3 g/l potassium dihydrogenphosphate, 1 g/l ammonium chloride, 250 mg/l magnesium sulfate heptahydrate (separately sterilized) and 4 mg/l vitamin B₁ (separately sterilized) (pH unadjusted) in a 5-1 fermentor, followed by culturing at 28°C with stirring (600 rpm) and aeration (2.5 l/minute). The pH was maintained at 7.0 with 14% (v/v) aqueous ammonia through the culturing.

Addition of a feed solution comprising 167 g/l glucose and 167 g/l peptone to the culture using a Perista pump was started 11 hours after the start of the culturing and was continued for 13 hours at a rate of 30 ml/hour.

Separately, Corynebacterium ammoniagenes ATCC 21170 was inoculated into 200 ml of a liquid medium comprising 50 g/l glucose, 10 g/l polypeptone (Daigo Eiyo Kagaku Co., Ltd.), 10 g/l yeast extract (Daigo Eiyo Kagaku Co., Ltd.), 5 g/l urea, 5 g/l ammonium sulfate, 1 g/l potassium dihydrogenphosphate, 3 g/l dipotassium hydrogenphosphate, 1 g/l magnesium sulfate heptahydrate, 0.1 g/l calcium chloride dihydrate, 10 mg/l ferrous sulfate heptahydrate, 10 mg/l zinc sulfate heptahydrate, 20 mg/l manganese sulfate tetra- to hexahydrate, 20 mg/l L-cysteine, 10 mg/l calcium D-pantothenate, 5 mg/l vitamin B₁, 5 mg/l nicotinic acid and 30 µg/l biotin (pH adjusted to 7.2 with sodium hydroxide) in a 2-1 Erlenmeyer flask with baffles, followed by rotary shaking culture at 220 rpm at 28°C for 24 hours. The obtained culture (20 ml) was inoculated into 2.5 1 of a liquid medium comprising 100 g/l glucose, 10 g/l polypeptone, 1 g/l potassium dihydrogenphosphate, 1 g/l dipotassium hydrogenphosphate, 1 g/l magnesium sulfate heptahydrate, 0.1 g/l calcium chloride dihydrate, 20 mg/l ferrous sulfate heptahydrate, 10 mg/l zinc sulfate heptahydrate, 20 mg/l manganese sulfate tetra- to hexahydrate, 15 mg/l β-alanine, 20 mg/l L-cysteine, 0.1 mg/l biotin, 2 g/l urea (separately sterilized) and 5 mg/l vitamin B₁ (separately sterilized) (pH adjusted to 7.2 with sodium hydroxide) in a 5-1 fermentor, followed by culturing at 32°C with stirring (600 rpm) and aeration (2.5 l/minute). The pH was maintained at 6.8 with concentrated aqueous ammonia through the culturing.

During the culturing, the culture was sampled and the glucose concentration in the supernatants obtained by centrifuging the samples was measured. When the glucose concentration in the supernatant became zero, 350 ml of the culture was aseptically recovered. The recovered culture was inoculated into 2.5 1 of a liquid medium comprising 180 g/l glucose, 10 g/l potassium dihydrogenphosphate, 10 g/l dipotassium hydrogenphosphate, 10 g/l magnesium sulfate heptahydrate. 0.1 g/l calcium chloride dihydrate, 20 mg/l ferrous sulfate heptahydrate, 10 mg/l zinc sulfate heptahydrate, 20 mg/l manganese sulfate tetra- to hexahydrate, 15 mg/l β-alanine, 1 g/l sodium glutamate, 20 mg/l L-cysteine, 0.1 mg/l biotin, 2 g/l urea (separately sterilized) and 5 mg/l vitamin B₁ (separately sterilized) (pH adjusted to 7.2 with sodium hydroxide) in a 5-1 fermentor, followed by culturing at 32°C with stirring (600 rpm) and aeration (2.5 l/minute). The pH was maintained at 6.8 with concentrated aqueous ammonia through the culturing. During the culturing, the culture was sampled and the glucose concentration in the supernatants obtained by centrifuging the samples was measured. The culturing was completed when the glucose concentration in the supernatant became zero.

The thus obtained cultures of Escherichia coli MM294/pCKG55 (500 ml) and Corynebacterium ammoniagenes ATCC 21170 (185 ml) were put into each of two 2-1 fermentors, and 80 ml of a 60% (w/v) glucose solution, 25 ml of 25% (w/v) magnesium sulfate heptahydrate and 160 ml of 25% (w/v) potassium dihydrogenphosphate were added thereto. To the resulting mixture were further added xylene and choline chloride to concentrations of 20 ml/l and 8.4 g/l (60 mM), respectively.

To one of the two fermentors (Pot 1) was added uracil (Sigma) to a concentration of 44 mmol/l and to the other (Pot 2) was added orotic acid (Sigma) to a concentration of 44 mmol/l. Each fermentor was allowed to stand at 32°C with stirring (800 rpm) and aeration (0.2 l/minute). The pH was maintained at 7.2 with 10 N sodium hydroxide. After 22 hours, the concentration of CDP-choline in the mixture was measured by HPLC. It was revealed that 12.1 g/l (24.8 mmol/l) of CDP-choline was formed in Pot 1 and 11.5 g/l (23.6 mmol/l) in Pot 2. A better result was obtained with use of uracil as a substrate than orotic acid.

### Industrial Applicability

- The present invention provides an industrially advantageous process for producing CDP-choline from choline or phosphorylcholine, or a salt thereof, and uracil.

## Claims

1. A process for producing CDP-choline, which comprises contacting a biocatalyst having the activity to form CDP-choline from choline or phosphorylcholine, or a salt thereof, and uracil with choline or phosphorylcholine, or a salt thereof, and uracil in an aqueous medium; allowing the CDP-choline to form and accumulate in the aqueous medium; and recovering CDP-choline from the aqueous medium;
and wherein the biocatalyst comprises cells selected from the group consisting of microorganisms belonging to the genera Escherichia or Corynebacterium, a culture of the cells or a treated matter of the cells;
and wherein the cells are transformants obtainable by introducing DNA encoding cytidine 5'- triphosphate synthetase having the activity to form cytidine 5' - triphosphate from uridine 5'-triphosphate, DNA encoding choline kinase having
the activity to form phosphorylcholine from choline, or DNA encoding cholinephosphate cytidyltransferase having the activity to form CDP-choline from cytidine 5' - triphosphate and phosphorylcholine.

2. The process according to Claim 1, wherein the microorganism belonging to the genus Corynebacterium is Corynebacterium ammoniagenes.

3. The process according to Claim 1, wherein the microorganism belonging to the genus Escherichia is Escherichia coli.

## Patentansprüche

1. Verfahren zum Herstellen von CDP-Cholin, welches umfaßt, daß man einen Biokatalysator, der die Aktivität zur Bildung von CDP-Cholin aus Cholin oder Phosphorylcholin oder einem Salz davon und Uracil besitzt, mit Cholin oder Phosphorylcholin oder einem Satz davon und Uracil in einem wäßrigen Medium in Kontakt bringt, das CDP-Cholin in dem wäßrigen Medium sich bilden und ansammeln läßt und CDP-Cholin aus dem wäßrigen Medium gewinnt,
und wobei der Biokatalysator Zellen, ausgewählt aus der Gruppe, bestehend aus Mikroorganismen, die zur Gattung Escherichia oder Corynebacterium gehören, eine Kultur der Zellen oder eine behandelte Substanz der Zellen umfaßt,
und wobei die Zellen Transformanten sind, die erhalten werden können durch Einbringen von DNA, die Cytidin-5'-triphosphatsynthetase codiert, welche die Aktivität zur Bildung von Cytidin-5'-triphosphat aus Uridin-6'-triphasphat besitzt, von DNA, die Cholinkinase codiert, welche die Aktivität zur Bildung von Phosphorylcholin aus Cholin besitzt, oder von DNA, die Cholinphosphatcytidyltransferase codiert, welche die Aktivität zur Bildung von CDP-Cholin aus Cytidin-5'-triphosphat und Phosphorylcholin besitzt.

2. Verfahren nach Anspruch 1, wobei der zur Gattung Corynebacterium gehörend Mikroorganismus Corynebacterium ammoniagenes ist.

3. Verfahren nach Anspruch 1, wobei der zur Gattung Escherichia gehörende Mikroorganismus Escherichia coli ist.

## Revendications

1. Procédé pour produire de la CDP-choline qui comprend le fait de mettre en contact un biocatalyseur ayant l'activité pour former de la CDP-choline à partir de choline ou de phosphorylcholine, ou d'un sel de celle-ci, et d'uracile avec la choline ou la phosphorylcholine, ou un sel de celle-ci, et l'uracile dans un milieu aqueux ; le fait de permettre à la CDP-choline de se former et de s'accumuler dans le milieu aqueux ; et le fait de récupérer la CDP-choline à partir du milieu aqueux ;
et où le biocatalyseur comprend des cellules choisies dans le groupe consistant en les micro-organismes appartenant aux genres Escherichia ou Corynebacterium, une culture des cellules ou un produit des cellules traité ;
et où les cellules sont des transformants pouvant être obtenus par introduction d'un ADN codant la cytidine 5'-triphosphate synthétase ayant l'activité pour former du cytidine 5'-triphosphate à partir d'uridine 5'-triphosphate, d'un ADN codant une choline kinase ayant l'activité pour former de la phosphorylcholine à partir de choline, ou d'un ADN codant une cholinephosphate cytidyltransférase ayant l'activité pour former de la CDP-choline à partirr de cytidine 5'-triphospshate et de phosphorylcholine.

2. Procédé selon la revendication 1 où le micro-organisme appartenant au genre Corynebacterium est Corynebacterium ammoniagenes.

3. Procédé selon la revendication 1 où le micro-organisme appartenant au genre Escherichia est Escherichia coli.
